# EUROPEAN PATENT SPECIFICATION

(11) **EP 0 268 743 B1**
(45) Date of publication and mention of the grant of the patent: **15.06.1994**
(21) Application number: 87110601.9
(22) Date of filing: 23.03.1984
(51) Int. Cl.: C12N 15/00, C12P 21/00, C12N 9/52, A61K 39/395

(54) **A process for the production of a protein**
Verfahren zur Herstellung eines Proteins
Procédé de production d'une protéine

(30) Priority: 25.03.1983 GB 8308234; 07.06.1983 WO PCT/GB83/00152; 12.10.1983 GB 8327345
(43) Date of publication of application: 01.06.1988
(62) Divisional of application: 84301995.1
(73) Proprietor: CELLTECH LIMITED, Slough Berkshire SL1 4DY (GB)
(72) Inventor: Lowe, Peter Anthony, Reading Berkshire RG6 2BN (GB); Marston, Fiona Angela Olinda, Reading Berkshire RG5 3PJ (GB); Angal, Sarojani, Reading Berkshire RG2 7EQ (GB); Schoemaker, Joyce Ann, Chiswick London W4 1EN (GB)
(74) Representative: Votier, Sidney David

(56) References cited:
- EP-A- 0 068 691
- WO-A-83/04418
- H.R. MAHLER et al.: "Biological Chemistry", second edition, 1971, pages 177-183, Harper & Row, New York, US
- CHEMICAL ABSTRACTS, vol. 87, no. 19, 7th November 1977, page 216, abstract no.147755e, Columbus, Ohio, US; D.M. MULVIHILL et al.: "Selective denaturation of milk coagulants in 5 M-urea"
- CHEMICAL ABSTRACTS, vol. 97, no. 19, 8th November 1982, page 528, abstract no.160800s, Columbus, Ohio, US; A. SUMI et al.: "Denaturation by guanidinehydrochloride of the Fc(t) and pFc' fragments of human immunoglobulin G"
- Aminosäuren, Peptide, Proteine by H. O. Jakubke und H. Jeschkeit, Weinheim 1982, pages 402-404
- Nature 285, 1980, pages 456-461

## Description

This invention relates to the field of protein production using recombinant DNA biotechnology. In particular it relates to a process for the recovery of a protein produced in an insoluble form by a host organism transformed with a vector including a gene coding for the protein.

There are now numerous examples of commercially valuable proteins which may be produced in large quantities by culturing a host organism capable of expressing heterologous genetic material. Once a protein has been produced by a host organism it is usually necessary to treat the host organism in some way, in order to obtain the desired protein. In some cases, such as in the production of interferon in Escherichia coli a lysis or permeabilisation treatment alone may be sufficient to afford satisfactory yields. However, some proteins are produced within a host organism in the form of insoluble protein aggregates which are not susceptible to extraction by lysis or permeabilisation treatment alone. It has been reported that a human insulin fusion protein produced in E.coli forms insoluble protein aggregates (see D.C. Williams et al (1982) Science 215 687-689).

A protein exists as a chain of amino acids linked by peptide bonds. In the normal biologically active form of a protein (hereinafter referred to as the native form) the chain is folded into a thermodynamically preferred three dimensional structure, the conformation of which is maintained by relatively weak interatomic forces such as hydrogen bonding, hydrophobic interactions and charge interactions. Covalent bonds between sulphur atoms may form intramolecular disulphide bridges in the polypeptide chain, as well as intermolecular disulphide bridges between separate polypeptide chains of multisubunit proteins, e.g. insulin. The insoluble proteins produced in some instances do not exhibit the functional activity of their natural counterparts and are therefore in general of little use as commercial products. The lack of functional activity may be due to a number of factors but it is likely that such proteins produced by transformed host organisms are formed in a conformation which differs from that of their native form. They may also possess unwanted intermolecular disulphide bonds not required for functional activity of the native protein in addition to intramolecular disulphide bonds. The altered three dimensional structure of such proteins not only leads to insolubility but also diminishes or abolishes the biological activity of the protein. It is not possible to predict whether a given protein expressed by a given host organism will be soluble or insoluble.

In our copending British patent application GB 2100737A we describe a process for the production of the proteolytic enzyme chymosin. The process involves cleaving a chymosin precursor protein produced by a host organism which has been transformed with a vector including a gene coding for the relevant protein. In the course of our work we discovered that the chymosin precursor proteins were not produced in their native form but as an insoluble aggregate. In order to produce a chymosin precursor in a native form which may be cleaved to form active native chymosin, the proteins produced by a host organism were solubilised and converted into their native form before the standard techniques of protein purification and cleavage could be applied.

In our copending published International patent application WO 83/04418 the methods used for the solubilisation of chymosin precursor proteins are described. In general the techniques described involve the denaturation of the protein followed by the removal of the denaturant thereby allowing renaturation of the protein. In one example the denaturant used is a compound such as urea or guanidine hydrochloride. When the insoluble precursor is treated with urea or guanidine hydrochloride it is solubilised. When the denaturant is removed, for example by dialysis, the protein returns to a thermodynamically stable conformation which, in the case of chymosin precursors, is a conformation capable of being converted to active chymosin.

The solubilised protein may be separated from insoluble cellular debris by centrifugation or filtration. The production of proteins from suitably transformed host organisms is potentially of great commercial value. The processes involved are of a type which may be scaled up from a laboratory scale to an industrial scale. However, where the protein produced is formed as an insoluble aggregate, potential complications in the process may increase the cost of production beyond a viable level. The solubilisation technique described above, whilst effective to solubilise such proteins, is relatively expensive and may represent a significant production cost.

We have discovered a generally applicable solubilisation process which, in its broadest aspect, does away with the requirement of relatively expensive reagents.

According to the present invention we provide a process for the production of a soluble native protein in which an insoluble form of the protein is produced by a an E.coli host transformed with a vector including a gene coding for the protein, wherein the insoluble form of the protein is reversibly denatured in an alkaline aqueous solution at a pH selected to promote dissociation of a group or groups of the protein inolved in maintaining the conformation of the protein and the protein is subsequently allowed to renature by reducing the pH of the solution below a pH effective to denature the protein to produce the soluble native form of the protein.

The use of an alkali solution to denature the insoluble protein reduces the reagent cost of the process. The pH is selected with reference to the protein to which the process is to be applied. In particular the pH is selected such that groups responsible for holding the protein in an unnatural conformation by means of intramolecular, or potentially in the case of a protein aggregate nonfunctional intermolecular bonds or forces are dissociated such that, when the pH is reduced, the protein refolds in the native conformation. The groups responsible for holding the protein in an unnatural conformation may be ionisable groups, in which case the pH is preferably selected to be compatible with pKa of the relevant ionizable group.

Studies in our laboratory have shown that intermolecular disulphide bonds exist in prochymosin aggregates produced in E.coli (Schoemaker et al (1984) submitted to PNAS). Native prochymosin is monomeric and contains three intramolecular disulphide bonds (Foltmann et al (1977) Proc. Natl. Acad. Sci. U.S.A. 74 pp 2321-2324). Six thiol groups per molecule are therefore available to form intermolecular and intramolecular bonds within the protein aggregate. Consequently, disulphide bonds must be broken and correctly reformed for denaturation/renaturation to successfully solubilise prochymosin. This may be achieved by using an alkaline aqueous solution of pH 10.7 (+0.5). The free thiol groups of cysteine have a pKa value of 10.46.

The term "insoluble" as used herein means in a form which, under substantially neutral conditions (for example pH in the range 5.5 to 8.5), is substantially insoluble or is in an insolubilised association with insoluble material produced on lysis of E.coli host cells. The insoluble product is either produced within the cells of the host organism in the form of insoluble relatively high molecular weight aggregates or may simply be associated with insoluble cell membrane material. The process permits the separation of solubilised protein from insoluble cellular debris.

Any suitable alkali may be used in the process, for example an aqueous solution of an alkali metal hydroxide such as NaOH or KOH, an aqueous buffer, or an aqueous solution of an organic base such as triethylamine.

Preferably the alkaline aqueous solution has a pH of from 9 to 11.5, most preferably from 10 to 11.

The treatment of an insoluble protein with an alkaline aqueous solution may not, in all cases, result in complete solubilisation of the protein. Since insoluble material is present at all times, a number of mass transfer effects may be important. It has been found that multiple extractions with alkali are more efficient than a single extraction even when large extraction volumes are used. This also has the advantage of minimising the time for which the solubilised protein is in contact with alkali. Preferably, therefore, one or more extractions of denatured protein are performed.

The insoluble protein may be a recombinant animal protein produced by the E.coli host. Examples of such proteins are immunoglobulin light and heavy chain polypeptides, foot and mouth disease antigens and thymosin and insulin proteins.

The E.coli host may be a naturally occuring organism or a mutated organism capable of producing an insoluble protein. Preferably, however, the host organism is an organism or the progeny of an organism which has been transformed using recombinant DNA techniques with a heterologous DNA sequence which codes for the production of a protein heterologous to the host organism and which is produced in an insoluble form. Examples of specific host organism strains include E.coli HB101, E.coli X1776, E.coli X2882, E.coli PS410, E.coli RV308 and E.coli MRC1.

The host organism may be transformed with any suitable vector molecule including plasmids such as ColE1, pCR1, pBR322, RP4 and phage λ DNA or derivatives of any of these.

Prior to treatment with the process of the present invention the host cells may be subjected to an appropriate lysis or permeabilisation treatment to facilitate recovery of the product. For example, the host organism may be treated with an enzyme, for example a lysozome, or a mechanical cell destructing device to break down the cells.

The process of the invention may then be employed to solubilise the insolubilised product and the resulting solution may be separated from solid cell material such as insoluble cell membrane debris. Any suitable method including filtration or centrifugation may be used to separate solution containing the solubilised protein from the solid cell material.

The present invention is now illustrated by way of the following Examples:

### Example 1

An experiment was conducted in which the solubilisation of insoluble methionine-prochymosin produced by E.coli cells transformed with vector pCT70 was achieved using alkaline denaturation. The preparation of the transformed E.coli cell line is described in detail in published British patent application GB2100737A.

Frozen E.coli pCT70 cells grown under induced conditions were suspended in three times their own weight of 0.05 M Tris-HCl pH 8, 1 mM EDTA, 0.1 M NaCl, containing 23 »g/ml phenylmethylsulphonylfluoride (PMSF) and 130 »g/ml of lysozyme and the suspension was incubated at 4°C for 20 minutes. Sodium deoxycholate was added to a final concentration of 0.5% and 10 »g of DNA ase 1 (from bovine pancreas) was added per gram of E.coli starting material. The solution was incubated at 15°C for 30 minutes by which time the viscosity of the solution had decreased markedly. The extract, obtained as described above, was centrifuged for 45 minutes at 4°C and 10000 x g. At this stage effectively all the methionine-prochymosin product was in the pellet fraction in insolubilised form, presumably as a result of aggregation or binding to cellular debris. The pellet was washed in 3 volumes of 0.01 M tris-HCl pH8, 0.1 M NaCl, 1 mM EDTA at 4°C. After further centrifugation, as above, the supernatant solution was discarded and the pellet resuspended in 3 volumes of alkali extraction buffer: 0.05 M K₂ HPO₄, 1 mM EDTA, 0.1 M NaCl, pH 10.7 and the suspension adjusted to pH 10.7 with sodium hydroxide. The suspension was allowed to stand for at least 1 hour (and up to 16 hours) at 4°C, the pH of the supernatant adjusted to 8.0 by addition of concentrated HCl and centrifuged as above. Methionine-prochymosin, representing a substantial proportion of the methionine-prochymosin originally present in the pellet, was found to be present in the supernatant in a soluble form which could be converted to catalytically active chymosin by acidification/neutralisation activation treatment substantially as described in published British patent application GB2100737A.

We further noted that re-extraction of the debris left after the first alkali extraction liberates an equivalent amount of prochymosin. Alkali extraction may be repeated to a total of 4-5 times with the liberation of approximately equivalent levels of prochymosin at each extraction.

### Example 2

An experiment was conducted in which the solubilisaton of an insoluble immunoglobulin light chain polypeptide produced by E.coli cells transformed with vector pNP3 was achieved using alkaline denaturation. The preparation of the transformed E.coli cell line is described in copending International patent application PCT/GB 84/ of even date herewith. E.coli cells transformed with the plasmid pNP3, containing a gene coding for the λ₁ light chain of the 4-hydroxy-3-nitrophenyl acetyl (NP) binding monoclonal antibody S43, were grown under inducing conditions. The cells were harvested and resuspended in 0.05 M TRIS pH 8.0, 0.233 M NaCl, 5% glycerol v/v containing 130 »g/ml of lysozyme and incubated at 4°C or room temperature for 20 minutes. Sodium deoxycholate was then added to a final concentration of 0.05% and 10 ug of DNA ase 1 (from bovine pancreas) was added per gm wet wt of E.coli. The solution was then incubated at 15°C for 30 minutes by which time the viscosity of the solution had decreased markedly. The resultant mixture was then centrifuged (at 10000 x g for 15 minutes for small volumes (1 ml) or 1 hour for larger volumes). Immunoprecipitation studies indicated that the λ light chain protein was present in the insoluble fraction rather than the soluble fraction.

In order to purify the recombinant light chain, the E.coli pellet fraction, obtained as described above, was resuspended in a pH 11.5 buffer comprising 40 mM K₂ HPO₄, 0.1 M NaCl and 1 mM EDTA. The suspension was allowed to stand for at least 1 hour (and up to 16 hours), centrifuged as above and the pH of the supernatant adjusted to 8.0 by addition of concentrated HCl. A substantial proportion of the light chain protein, orginally present in the pellet was found to be present in the supernatant in a soluble form.

## Claims

1. A process for the production of a soluble native protein in which an insoluble form of the protein is produced by an E.coli host transformed with a vector including a gene coding for the protein, wherein the insoluble form of the protein is reversibly denatured in an alkaline aqueous solution at a pH selected to promote dissociation of a group or groups of the protein involved in maintaining the conformation of the protein, and the protein is subsequently allowed to renature by reducing the pH of the solution below a pH effective to denature the protein to produce the soluble native form of the protein.

2. A process according to claim 1 wherein the alkaline aqueous solution has a pH from 9 to 11.5.

3. A process according to claim 1 or 2 wherein the insoluble form of the protein is present in conjunction with debris derived from the host organism which is insoluble in alkaline aqueous solution and wherein one or more extractions of denatured protein are performed.

## Patentansprüche

1. Verfahren zur Herstellung eines löslichen nativen Proteins, bei welchem eine unlösliche Form des Proteins von einem E. coli-Wirt produziert wird, der mit einem Vektor transformiert wurde, der ein für das Protein codierendes Gen enthält, wobei die unlösliche Form des Proteins in einer alkalischen wässerigen Lösung bei einem pH-Wert reversibel denaturiert wird, der im Hinblick auf die Begünstigung der Dissoziation einer Gruppe oder von Gruppen des Proteins, die an der Aufrechterhaltung der Konfiguration des Proteins beteiligt ist bzw. sind, ausgewählt wurde, und wobei anschließend die Renaturierung des Proteins ermöglicht wird, indem der pH-Wert der Lösung unter einen Wert abgesenkt wird, üs der die Denaturierung des Proteins bewirkt, um dadurch die lösliche native Form des Proteins zu gewinnen.

2. Verfahren nach Anspruch 1, bei welchem die alkalische wässerige Lösung einen pH-Wert von 9 bis 11,5 besitzt.

3. Verfahren nach Anspruch 1 oder 2, bei welchem die unlösliche Form des Proteins in Verbindung mit aus dem Wirtsorganismus stammenden Zellbruchstücken vorliegt, die in der alkalischen wässerigen Lösung unlöslich sind, und bei welchem eine oder mehrere Extraktionen des denaturierten Proteins durchgeführt werden.

## Revendications

1. Procédé de production d'une protéine naturelle soluble, dans lequel une forme insoluble de la protéine est produite par un hôte E.coli transformé avec un vecteur incluant un gène codant pour la protéine, dans lequel la forme insoluble de la protéine est dénaturée de façon réversible dans une solution aqueuse basique à un pH choisi pour favoriser la dissociation d'un groupe ou de groupes de la protéine impliquée en maintenant la conformation de la protéine, et on laisse ultérieurement la protéine se renaturer en abaissant le pH de la solution au-dessous d'un pH efficace pour dénaturer la protéine en vue de produire la forme naturelle soluble de la protéine.

2. Procédé selon la revendication 1, dans lequel la solution aqueuse basique possède un pH de 9 à 11,5.

3. Procédé selon la revendication 1 ou 2, dans lequel la forme insoluble de la protéine est présente conjointement avec des débris dérivés de l'organisme hôte qui est insoluble dans la solution aqueuse basique et dans lequel une ou plusieurs extractions de protéine dénaturée sont réalisées.
